# EUROPEAN PATENT SPECIFICATION

(11) **EP 1 660 444 B1**
(45) Date of publication and mention of the grant of the patent: **12.11.2014**
(21) Application number: 04788633.8
(22) Date of filing: 07.09.2004
(51) Int. Cl.: C07D 487/22, C07D 401/14, C07D 403/14, C07D 487/04, C07F 15/00, A61P 35/00, A61K 31/00

(54) **METAL COMPLEXES OF N-HETEROCYCLIC CARBENES AS RADIOPHARMACEUTICALS AND ANTIBIOTICS**
METALLKOMPLEXE VON N-HETEROCYCLISCHEN CARBENEN ALS RADIOPHARMAZEUTIKA UND ANTIBIOTIKA
COMPLEXES METALLIQUES DE CARBENES N-HETEROCYCLIQUES UTILES COMME PRODUITS RADIOPHARMACEUTIQUES ET ANTIBIOTIQUES

(30) Priority: 05.09.2003 US 500737 P
(43) Date of publication of application: 31.05.2006
(73) Proprietor: The University of Akron, Akron Ohio 44325 (US)
(72) Inventor: YOUNGS, Wiley, J., Akron, OH 44321 (US); TESSIER, Claire, A., Akron, OH 44321 (US); GARRISON, Jered, Columbia, MO 65202 (US); QUEZADA, Carol, Barbeton, OH 44203 (US); MELAIYE, Abdulkareem, Akron, OH 44310 (US); PANZNER, Matthew, Akron, OH 44313 (US); DURMUS, Semih, Akron, OH 44304 (US); AYSEGUL, Kascatan-Nebioglu, Akron, OH 44305 (US)
(74) Representative: Von Kreisler Selting Werner - Partnerschaft von Patentanwälten und Rechtsanwälten mbB
(86) International application number: PCT/US2004/029285
(87) International publication number: WO 2005/023760

(56) References cited:
- US-B1- 6 288 197
- US-B2- 6 794 327
- US-B2- 6 919 448
- YOUNGS W. J. ET AL: "Formation of N-Heterocyclic Complexes of Rhodium and Palladium from a Pincer Silver(I) Carbene Complex" ORGANOMETALLICS, ACS, WASHINGTON, DC, US, vol. 22, no. 22, 26 March 2003 (2003-03-26), pages 1979-1982, XP002457597 ISSN: 0276-7333
- GARRISON J C ET AL: "Synthesis and structural characterization of a silver complex of a mixed-donor N-heterocyclic carbene linked cyclophane" CHEMICAL COMMUNICATIONS 21 SEP 2001 UNITED KINGDOM, no. 18, 21 September 2001 (2001-09-21), pages 1780-1781, XP002457598 ISSN: 1359-7345
- DANOPOULOS A. A. ET AL: "Chelating and "pincer" dicarbene complexes of palladium; synthesis and structural studies" JOURNAL OF THE CHEMICAL SOCIETY, DALTON TRANSACTIONS, CHEMICAL SOCIETY. LETCHWORTH, GB, 30 January 2003 (2003-01-30), pages 1009-1015, XP002457599 ISSN: 1472-7773
- ALCALDE E ET AL: "Open-Chain Dications and Betaines with Imidazolium Molecular Motifs: Synthesis and Structural Aspects" CHEMISTRY - A EUROPEAN JOURNAL, VCH PUBLISHERS, US, 2002, pages 1221-1231, XP002457600 ISSN: 0947-6539
- NIELSEN ET AL: "A pyridine bridged dicarbene ligand and it's silver(I) and palladium(II) complexes: synthesis, structures and catalytic applications" INORGANICA CHIMICA ACTA, X, XX, vol. 327, 2002, pages 116-125, XP002457601 ISSN: 0020-1693
- GARRISON J C ET AL: "Synthesis and structural characterization of a [Ag4]<4+> cluster stabilized by a mixed-donor N-heterocyclic carbene linked cyclophane and the first reported synthesis of a N-heterocyclic carbene complex in water", JOURNAL OF ORGANOMETALLIC CHEMISTRY, ELSEVIER-SEQUOIA S.A. LAUSANNE, CH, vol. 673, no. 1-2, 30 April 2003 (2003-04-30), pages 1-4, XP004423343, ISSN: 0022-328X, DOI: 10.1016/S0022-328X(03)00151-7
- H.J Klasen: "A historical review of the use of silver in the treatment of burns. II. Renewed interest for silver", Burns, vol. 26, no. 2, 1 March 2000 (2000-03-01), pages 131-138, XP055055583, ISSN: 0305-4179, DOI: 10.1016/S0305-4179(99)00116-3

## Description

### BACKGROUND OF THE INVENTION

This invention relates to metal-containing, therapeutic, antimicrobial, and antifungic compounds. More particularly, this invention relates to metal complexes of N-heterocyclic carbenes and their use as antimicrobial agents, antifungic agents and radiopharmaceutical compositions.

Silver has long been used for its antimicrobial properties. This usage predates the scientific or medical understanding of its mechanism. For example, the ancient Greeks and Romans used silver coins to maintain the purity of water. Today silver is still used for this same purpose by NASA on its space shuttles. Treatment of a variety of medical conditions using silver nitrate was implemented before 1800. A 1% silver nitrate solution is still widely used today after delivery in infants to prevent gonorrheal ophthalmia. Since at least the later part of the nineteenth century, silver has been applied in a variety of different forms to treat and prevent numerous types of bacteria related afflictions.

Other treatments, such as the application of silver foil to post surgical wounds to prevent infection survived as a medical practice into the 1980's in Europe, and silver nitrate is still used as a topical antimicrobial agent. In the 1960's the very successful burn treatment silver complex, silver sulfadiazine, shown in formula 1 below, was developed. Commercially known as Silvadene® Cream 1%, this complex has remained one of the most effective treatments for preventing infection of second and third degree bums. Silver sulfadiazine has been shown to have good antimicrobial properties against a number of gram-positive and gram-negative bacteria. It is believed that the slow release of silver at the area of the superficial wound is responsible for the process of healing. Studies on surgically wounded rats have shown the effectiveness of both silver nitrate and silver sulfadiazine to aid in the healing process. By using these common silver antimicrobial agents, inflammation and granulation of wounds were reduced, although the complete mechanism for these phenomena is not understood.

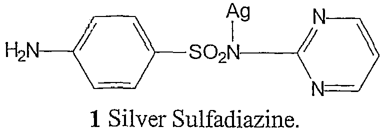

Recently developed silver-coating techniques have lead to the creation of a burn wound dressing called Acticoat. The purpose of this dressing is to avoid adhesion to wounds while providing a barrier against infection. Some clinical trials have also demonstrated the ease of removal of the dressing in contrast to conventional wound dressings treated with silver nitrate. Acticoat has shown an increase in antibacterial function over both silver nitrate and silver sulfadiazine. Acticoat is made up of nanocrystalline silver particles. Antibiotic-resistant strains have developed to both silver nitrate and silver sulfadiazine but not to nano-crystalline silver. The broader range of activity of nanocrystalline silver is apparently due to the release of both silver cations and uncharged silver species. Due to the continuing emergence of antibiotic resistant strains of infectious agents, a need exists for novel antibiotics.

Metal compounds have also played a significant role in other therapeutic applications. One example of the usefulness of the metals can be seen in the field of radiopharmaceuticals. The use of radiation therapy to destroy tumor cells is well known, but tumors can reappear after therapy. Hypoxic cells within the tumor are 2.5 to 3 times more resistant to X-ray radiation than other tumor cells. For this reason, these cells are more likely to survive radiation therapy or chemotherapy and lead to the reappearance of the tumor. Targeting of radionuclides to hypoxic cells will serve as a method to visualize them.

Complexes of y-ray emitters such as ⁹⁹Tc are extremely useful as imaging agents, and therapeutic radiopharmaceuticals like ⁸⁹Sr, ¹⁵³Sm, ¹⁸⁶Re and ¹⁶⁶Ho are important in the treatment of bone tumors. Rh-105 emits a gamma ray of 319 keV (19%) that would allow in vivo tracking and dosimetry calculations. Many more radioactive nuclei can be harnessed by using the entire periodic table to construct diagnostic or therapeutic agents.

The usefulness of complexes of radioactive metals is highly dependent on the nature of the chelating ligand. A successful metal drug must both target a specific tissue or organ as well as rapidly clear from other tissues. In addition, for both imaging and tumor treatment, the target organ or tissue must have optimal exposure to the radiopharmaceutical. Therefore, there is a need for novel ligand systems designed to bind radioactive metals.

J. C. Garrison et al. (J. Org. Chem. 2003, 673, 1-4) disclose silver complexes of multidentate N-heterocyclic carbene macrocycles for use in chemotherapy.

H. J. Klasen (Burns 2000, 26, 131-138) provides a review of the use of silver in the treatment of burns. In particular, silver compounds are disclosed that are able to prevent infections due to growth of pathogenic microorganisms and their penetration into the body of patients.

### SUMMARY OF THE INVENTION

While metal complexes of some N-heterocyclic carbenes have been previously known, it has not been recognized that silver complexes of N-heterocyclic carbenes will act as antimicrobial agents. It has likewise not been recognized that complexes of N-heterocyclic carbenes and radioactive metals may be used as radiopharmaceuticals. Strongly chelating ligands, such as the pincer N-heterocyclic carbenes, described herein, can provide an alternate, more advantageous route for the generation of radiopharmaceutical complexes.

Accordingly, the present invention relates to a pharmaceutical composition of a silver complex of an N-heterocyclic carbene characterized in that the silver complex of an N-heterocyclic carbene is represented by the formula: 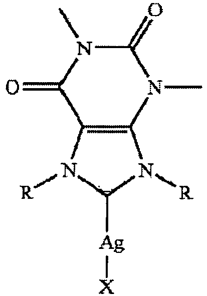 where each R is independently selected from a hydrogen, a C₁ to C₁₂ alkyl, a C₁ to C₁₂ substituted alkyl, a C₁ to C₁₂ alkoxy, a cycloalkyl having up to 12 carbon atoms, a substituted cycloalkyl having up to 12 carbon atoms, an alkenyl having up to 12 carbon atoms, a cycloalkenyl having up to 12 carbon atoms, a substituted cycloalkenyl having up to 12 carbon atoms, an alkynyl having up to 12 carbon atoms, an aryl having up to 12 carbon atoms, a substituted aryl having up to 12 carbon atoms, an arylalkyl having up to 12 carbon atoms, an alkylamine having up to 12 carbon atoms, an substituted alkylamine having up to 12 carbon atoms, an alkylpentose phosphate having up to 12 carbon atoms, a phenol having up to 12 carbon atoms, an ester having up to 12 carbon atoms, an alkylaryl having up to 12 carbon atoms, a pyrrole having up to 12 carbon atoms, a pyridine having up to 12 carbon atoms, or a thiophene having up to 12 carbon atoms, and where X is selected from a halide, a carbonate group, an acetate group, a phosphate group, a hexafluorophosphate group, a tetrafluoroborate group, a nitrate group, a methylsulfate group, a hydroxide or sulfate.

### DETAILED DESCRIPTION OF THE INVENTION

In this specification and the appended claims, the singular forms "a," "an," and "the" include plural reference unless the context clearly dictates otherwise. Unless defined otherwise, all technical and scientific terms used herein have the same meaning as commonly understood to one of ordinary skill in the art to which this invention pertains.

The present invention includes metal N-heterocyclic carbene complexes that can be made from N-heterocyclic carbene precursors, the imidazolium salts. The imidazolium salts can readily be reacted with silver(I) oxide in suitable solvent to obtain the silver-N-heterocyclic carbene complexes.

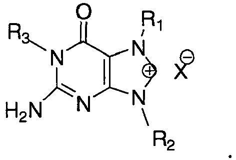

The imidazolium cation used in the present invention is represented by formula 51: 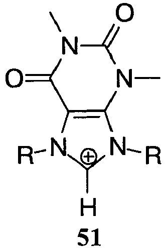 which can be used for the formation of monodentate N-heterocyclic carbene silver complex having formula 56. The carbene silver complex shown in formula 56 can be synthesized by the interaction of the imidazolium precursor 51 with a silver oxide: 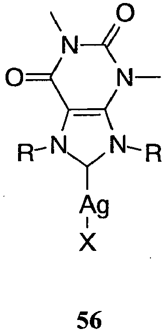 wherein R is defined as above.

The X⁻ counter anion may be from the group consisting of halides, carbonate, acetate, phosphate, hexafluorophosphate, tetrafluoroborate, nitrate, methylsulfate, hydroxide and sulfate.

Xanthines have been used for a number of years as bronchodilators for the treatment of airway obstructions in cystic fibrosis patients. Because xanthines contain imidazole rings we assumed it should be possible to alkylate them to form imidazolium cations and eventually silver carbene complexes. Because of their use as bronchodilators we also assumed that their methylated derivatives would be relatively nontoxic. Probably the most well know of the xanthines is caffeine 95. We have investigated the alkylation of caffeine to form methylated caffeine and the formation of silver carbene complexes using caffeine as the carbene precursor. Methylated caffeine has proven to be even less toxic than caffeine.

The methyl sulfate salt of methylated caffeine, 1,3,7,9-tetramethylxanthanium, 96a is given by the reaction of caffeine 95 with dimethyl sulfate in nitrobenzene. Anion exchange using NH₄PF₆ in water results in 96b.

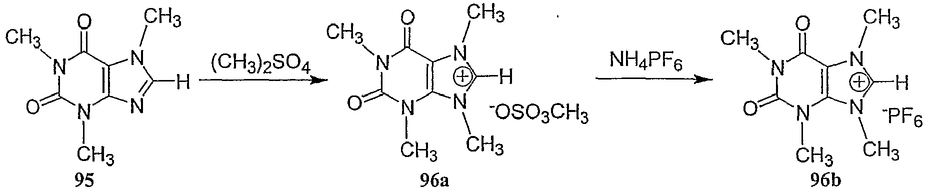

Ligand 96a is water soluble and reacts with Ag₂O in water to give complex 97a. 97a is stable in water for five days. The lack of C-¹⁰⁷ Ag and C-¹⁰⁹Ag couplings suggests fluxional behavior on the ¹³C NMR timescale as observed with many silver(I) complexes. Similarly, 96b reacts with Ag₂O in DMSO to form 97b, which has been structurally characterized by X-ray crystallography. The thermal ellipsoid plots (TEP) of the cationic portions of 96b and 97b are shown below.

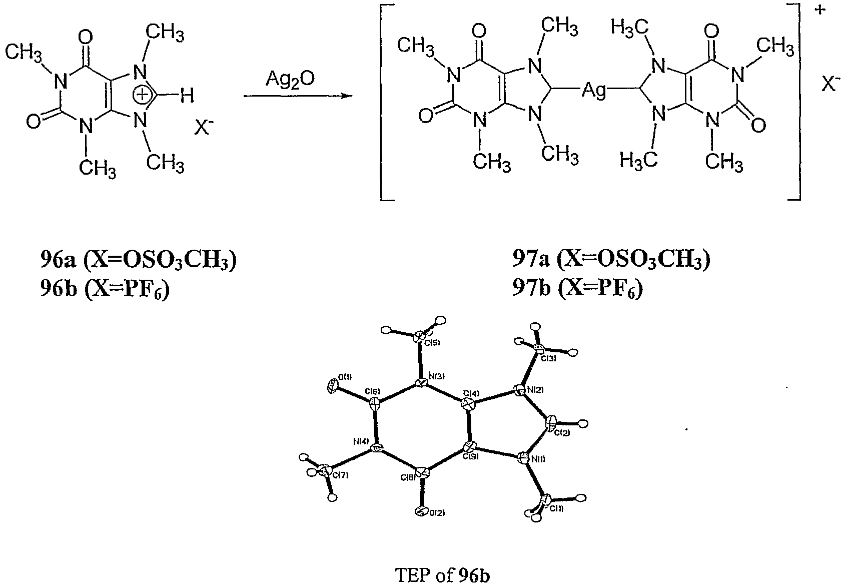

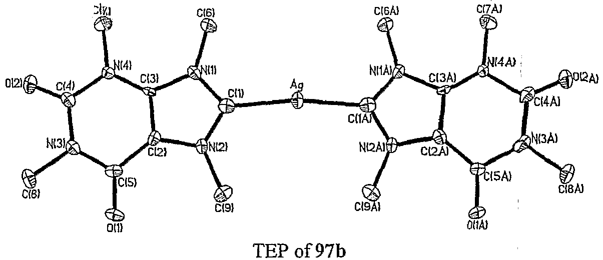

Caffeine, 1,3,7-trimethylxanthine, is one of the xanthine derivatives that are generally used in medicines as diuretics, central nervous system stimulants and inhibitors of cyclic adenosine monophosphate (c-AMP) phosphodiesterase. 1,3,7,9-tetramethylxanthinium iodide (methylated caffeine), an imidazolium salt, was synthesized using modified literature procedures and characterized by ¹H, ¹³C NMR, mass spectrometry and X-ray crystallography.

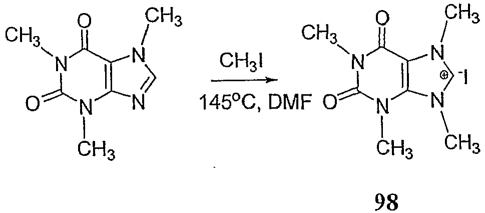

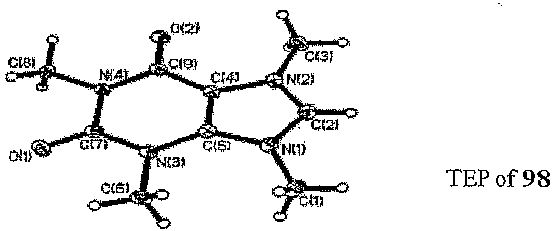

The reaction of two equivalent of 1,3,7,9-tetramethylxanthinium iodide with three equivalent of silver(I) oxide in methanol at room temperature gives compound 99.

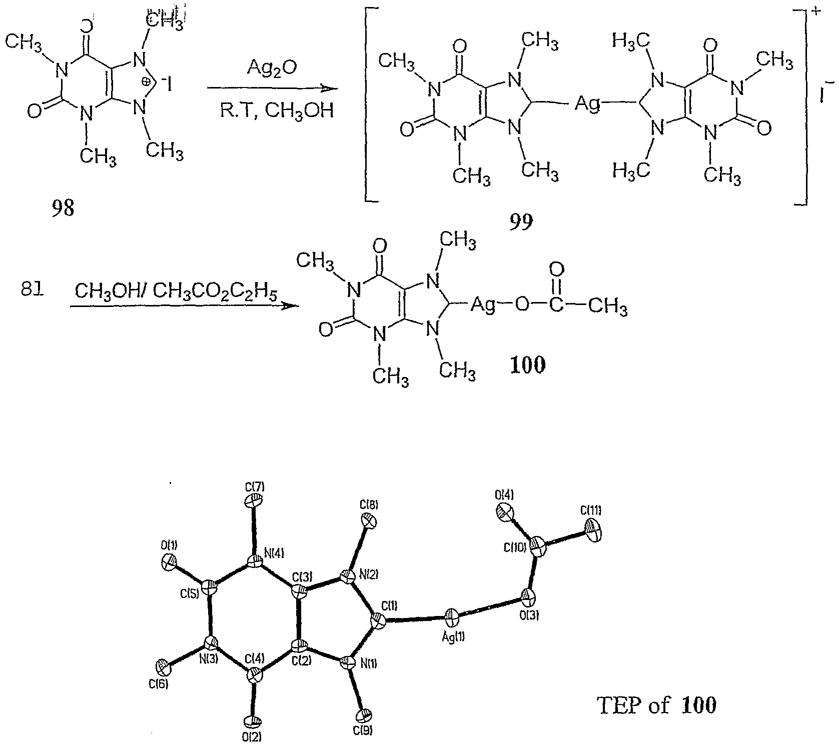

The crystallization of 99 in a mixture of methanol and ethyl acetate gives compound 100, a colorless crystal, soluble in water and air stable. Compounds 99 and 100 were characterized by ¹H, ¹³C NMR, and mass spectrometry. X-ray crystallography was used to confirm the molecular structure of 100 with the thermal ellipsoid plot show above. The antimicrobial properties of 100 have been evaluated using both the filter disk test and the standard MIC technique. Compound 100 was found to have effective antimicrobial activity on *S. aureus, P. areguinosa,* and *E*. *coli.* The dose-response effect on compound 98 was assessed to determine the toxicity of the compound on rats. The toxicity studies, is a standard protocol used to determine the lethal dose required to kill half (LD 50) of the animal (rats). The LD 50 assessment on compound 98 was 2.37 g per Kg of rat. The protocol used in this study was approved by the Institutional Animal Care and Use Committee (IACUC), University of Akron.

The delivery methods for administering an effective amount of transition metal complexes of N-heterocyclic carbenes for *in-vitro* and *in-vivo* medicinal application consist of aerosol, biodegradable polymers, polymeric micelles, hydrogel types materials, dendrimers, and modified C-60 fullerenes.

The present disclosure also relates to NHC salt 108 and the corresponding silver complex 106. Although said compounds are not according to the present invention the following paragraphs have been included for explanatory reasons to improve the intelligibility of the present invention.

The imidazolium (NHC) cyclophane gem-diol salt 108 can be prepared by reacting 2,6-bis(imidazolemethyl)pyridine with 1,3-dichloroacetone as shown below in Eq. 2. The formation of salt 108 as a gem-diol in preference to the carbonyl form is not expected with electron withdrawing groups present. Without being bound to theory, it is believed that the formation of salt 108 as a gem-diol proceeded by acid-catalyzed process with the solution observed to be slightly acidic having a pH range of 5-6.

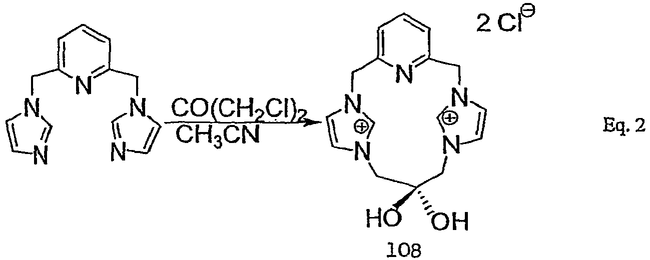

The ¹H NMR spectra showed the presence of *gem* O-H as a broad peak at 7.65ppm, and the absence of C=O in salt 108 was observed in both ¹³C NMR and IR spectroscopy. The O-H stretching vibration was observed at 3387 cm⁻¹, while the C-O stretching at 1171 cm⁻¹ and ¹³C NMR chemical shift at 91 ppm. The x-ray crystallography further provided the evidence and structure of 108 as shown in the following figure:

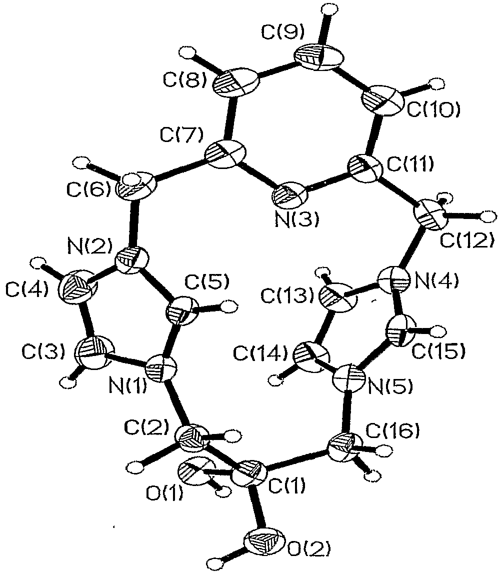

TEP of salt 108 with the thermal ellipsoid drawn at 50% probability level. The counter anions are omitted for clarity.

The combination ofsilver(I) oxide with salt 108 in methanol according to the reaction scheme illustrated in Eq. 3 results in complex 106 as an air and light stable yellow solid in high yield, confirmed by the loss of the imidazolium proton at 9.35 ppm of the ¹H NMR spectra. The proton NMR of complex 106 showed a broad signal with complicated peaks that are not easily assigned. Again, without being bound to theory, this may be due to the fluxional behavior of the

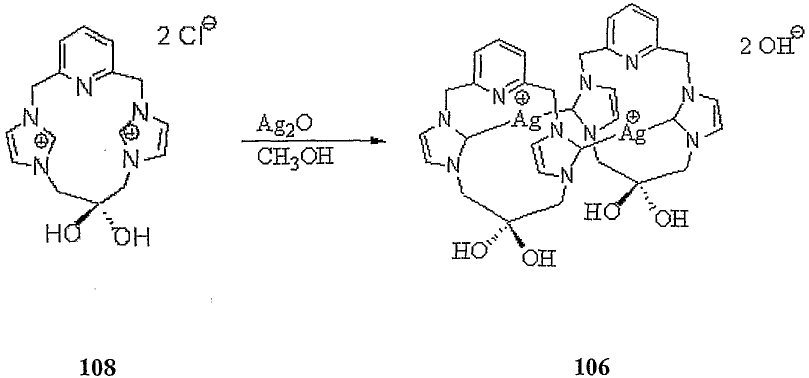

The shift in the resonance signal of the imidazole carbon (NCN) from 138 ppm to downfield of the ¹³C NMR spectra at 184 and 186 ppm shows the rare coupling of the Ag-C bond. The large value of the Ag-C coupling constant (*J*_{AgC} = 211 Hz) observed agreed with the reported range of 204 Hz - 220 Hz for ¹⁰⁹Ag nuclei coupling. ¹⁰⁹Ag coupling is commonly observed due to its higher sensitivity compared to the ¹⁰⁷Ag. The x-ray crystallography confirms the structure of complex 106, which is shown in formula II, with bond distances of Ag1-C15 = 2.085(5) Å, Ag1-C31 = 2.077(5) Å, Ag2-C5 = 2.073(5) Å and Ag2-C21 = 2.072 Å. A weak Agl...Ag2 interaction was observed with a bond length of 3.3751(10) A, longer than the commonly reported Ag-Ag bond range of 2.853-3.290 Å, but shorter than the Van der waals radii for Ag....Ag of 3.44 Å. In silver metal the Ag-Ag bond distance is known to be 2.888 Å. The C-Ag-C bond angles are almost linear with C15-Ag1-C31 bond angle of 175.20(18)° and C21-Ag2-C5 bond angle of 170.56(18)°.

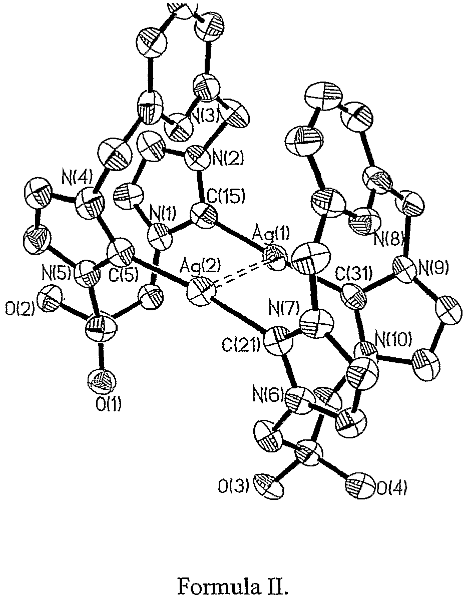

Thermal ellipsoid plot of complex 106 with the thermal ellipsoid drawn at 50% probability level. The counter anions are omitted for clarity.

The electrospun fibers from Tecophilic® and silver complex were characterized by transmission electron microscopy (TEM) and scanning electron microscopy (SEM). No obvious phase separation was observed in as-spun fibers, shown in Figure 3, which indicated a generally-uniform mixing of Tecophilic® and silver complex. The thickness of the fiber mat was measured by scanning electron microscopy (SEM) with pure Tecophilic® (100 micron), 25:75 silver complex 106/Tecophilic® (30 microns) and 75:25 complex 106/Tecophilic® (60 microns) respectively. The encapsulation of complex 106 by polymer retards the quick decomposition of silver complex into silver ions or particles in an aqueous media. The formation of silver particles at nanometer scale has been observed in the polymer matrix, when the electrospun fiber is exposed to water. Transmission electron microscopy studies showed that the activation of nano-silver particles in the fiber is a process that occurs gradually over a period of time. By exposing the as-spun fibers to water, complex 106 decomposed and release silver ions which aggregated into silver particles at nano-scale measurement. The formation of aggregates of silver particles has been observed within 30 minutes of exposure to water vapor (as shown in Figure 4). The aggregation of the silver ions in the presence of water, with the aggregate adsorbed on the surface of the fibers is considered to be a simplified mechanism by which the fiber mat releases the active form(s) of the silver for its antimicrobial activity. The fiber of complex 106 is stable in light and air for months, but sensitive to an environment with very high humidity.

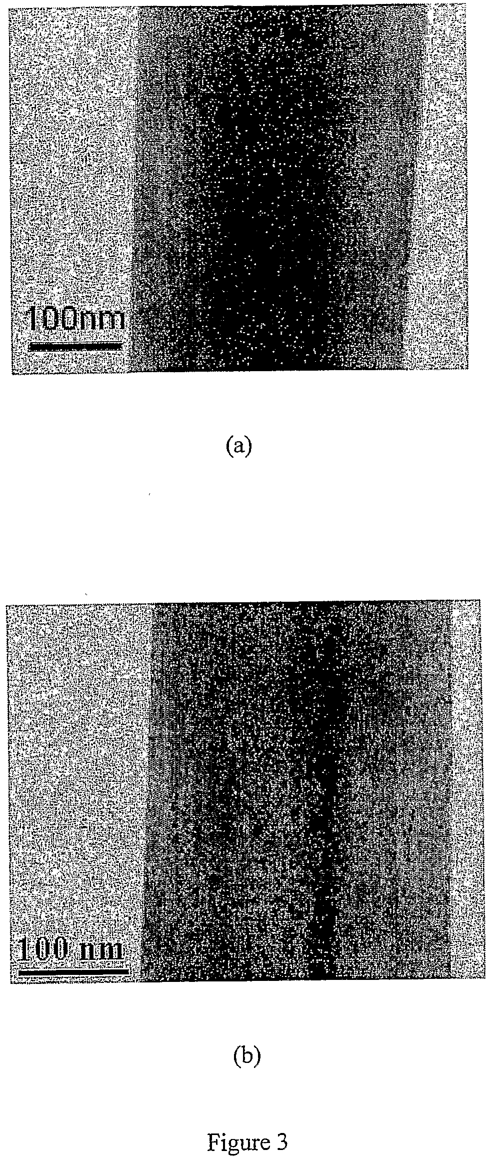

Electrospun fibers prepared from a mixture of 106 and Tecophilic® at a weight ratio of 25 to 75. (a) As-spun fiber (b) Silver particles formed by exposing the as-spun fiber to water.

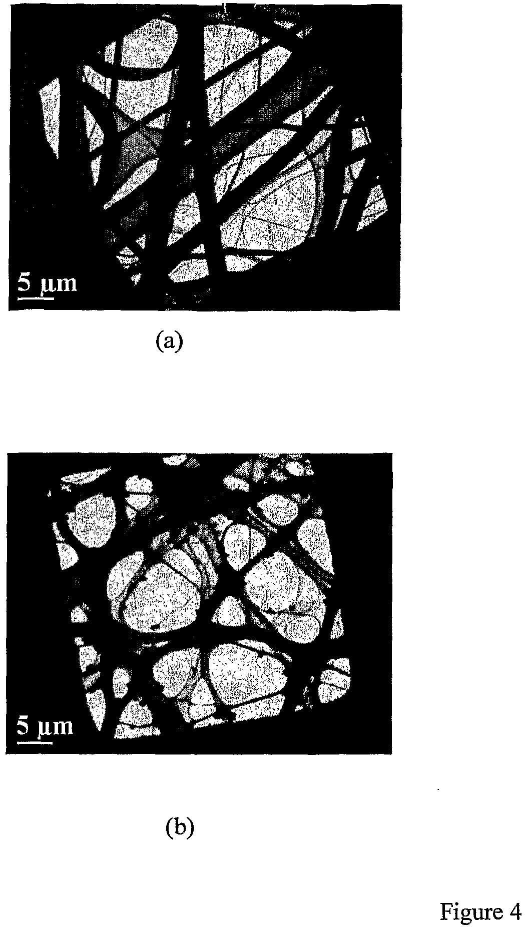

TEM images showing the release of silver particles by exposing fibers of complex 106 and Tecophilic® (weight ratio 50:50) to water vapor environment; (a) as-spun fiber, (b) fibers in water vapor environment for 65 hour.

### Bactericidal Effect

Using a modified Kirby Bauer technique mats of electrospun Tecophilic® fiber encapsulating complex 106 and pure electrospun Tecophilic® fiber as control were placed on a lawn of organism in an agar plate and incubated overnight at 35 °C. The inocula used were both Gram positive and Gram negative prokaryotes (*Escherichia coli, Pseudomonas aeruginosa,* and *Staphylococcus aureus*) of clinical interest. The fungi used were *Candida albicans, Aspergillus niger*, and *Saccharomyces cerevisiae*. The bactericidal activity showed a clear zone of inhibition within and around the fiber mat after an overnight incubation of the agar plate at 35 °C. The fungicidal activity was observed after 48 hrs of incubation at 25 °C. Pure Tecophilic® fiber mat as control showed no growth inhibition (See figure 5). No obvious difference was observed in the diameter of the cleared zone of inhibition around the fiber mat when the composition of the fiber mat was changed from 75 % of complex 106 and 25 % Tecophilic® to 25% of complex 106 and 75% Tecophilic®. The diameter of the zone of inhibition for the 75 complex 106/tecophilc®) fiber mat is 4.00mm while that of 25% (complex 106/tecophilc®) is 2.00 mm. The difference in diameter of the zone of inhibition between the two types of fiber mat has no linear relationship with the amount of silver (3:1 ratio) present in the two fiber mats. These result further shows the limitation of the Kirby Bauer technique as a quantitative tool to determine the antimicrobial activity of drugs. The diffusing ability of the silver ions might have been limited by the formation of secondary silver compounds. Ionic silver is known to undergo ligand exchange reactions with biological ligands such as nucleic acids, proteins, and cell membranes.

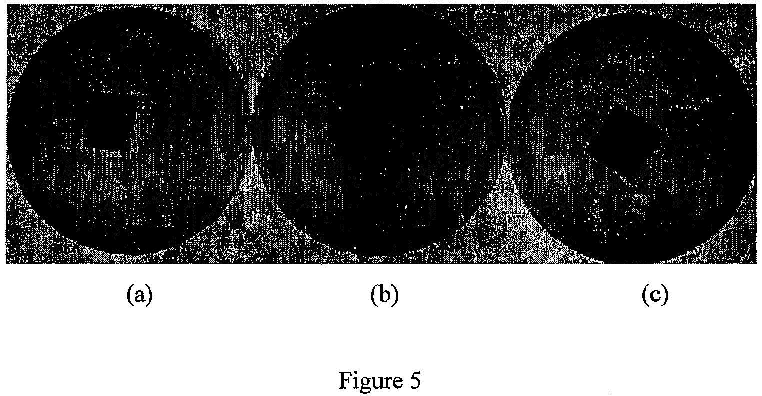

Susceptibility test of the fiber mat encapsulating complex 106, with bactericidal activity compared to pure Tecophilic® fiber mat. (a) complex 106/Tecophilic® (25:75) (b) Pure Tecophilic® (c) complex 106/Tecophilic® (75:25)

Deposition of a few silver particles was observed at the bottom of a test tube when a piece of the fiber mat was placed in 5 ml of distilled water and exposed to light for 4 days. The leaching of the silver particles from the fiber mat surfaces to the solution occurred gradually over time. The release of nano-silver particles from the as-spun mats of complex 106 into an aqueous medium lead to the investigation of the kinetics of kill (bactericidal activity) of the as-spun fiber mat of complex 06 with respect to time by comparing it with silver nitrate and silver sulfadiazine 1% cream or silvadene (SSD), a clinical drug widely in use. Both types of the fiber mat composition 75:25 (amount of Ag = 424 µg/mL) and 25:75 (amount of Ag = 140 µg/mL) used in this study showed a faster kill rate than SSD (amount of Ag = 3020 µg/mL). Silver nitrate (0.5 %) with 3176 µg/mL of Ag showed about the same kill rate as complex 106/tecophilic 75: 25 (Ag = 424 µg/mL) at a silver concentration 8 fold lower than silver nitrate (see figure 6). Bactericidal activity of the silver compounds is faster on *P. aeruginosa* than on *S. aureus.* The fiber mats killed bacteria faster better than silvadene.

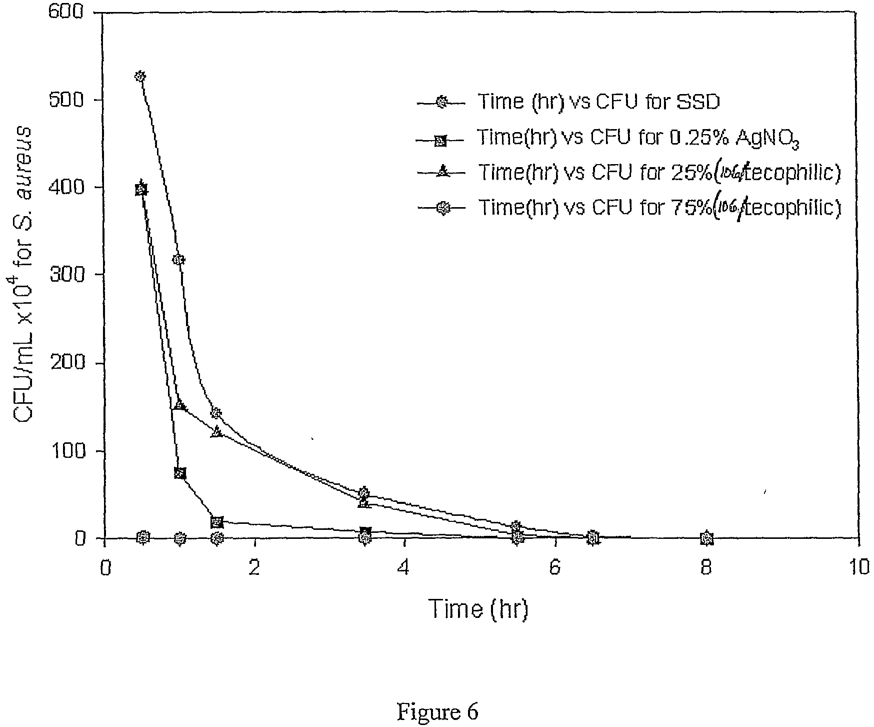

The Plot of CFU (colony forming unit) versus Time (hours) of the silver compounds on *S*. *aureus,* expresses the kinetic of the bactericidal activity for each of the silver compounds tested.

The time dependence of the bacteriostatic and bactericidal activities of the as-spun mat of complex 106 as a function of the volume of organism inoculated was examined. The fiber mats of complex 106 showed an effective bactericidal activity on *P. aeruginosa, E.coli* and *S*. *aureus* for over a week with daily inoculation (25µL) of freshly grown organism. This is an indication that the as-spun fiber mat sustained the continuous release of active silver species over a long period of time. Pure Tecophilic® mat as control showed no antimicrobial activity within 24hrs of incubation. The as-spun mat of complex 106 with the 75% complex 106/tecophilic composition showed better bactericidal effect on *P. aeruginosa* than the 25% complex 106/tecophilic for over 2 weeks after inoculating with over 200µL (2 x 10⁷) of freshly grown organism. Bacteriostatic activity was observed for *S*. *aureus* and *E. coli* after 10 days of the daily streaking of the LB broth solution on an agar plate. Visual inspection of the incubated solutions showed no growth of the organism.

The bactericidal activity of 108, complex 106 and AgNO₃ in aqueous LB broth was studied using the minimum inhibitory concentration (MIC) test. There was generally no difference in the bactericidal activity and MIC of complex 106 and AgNO₃ after 24 hrs of incubation as shown in Table 5. However, after 48 hrs of incubation, silver nitrate showed a better antimicrobial activity at a concentration 2 fold lower than complex 106 (838 µg/mL).

**Table 5. MIC result comparing the activity of AgNO₃ and complex 106, with both having about the same amount of silver. DF is the dilution factor (1 mL). + = growth, - = no growth. The amount of silver (µg) per mL for each compound was calculated as (molecular mass of Ag/formula wt of compound) x wt %.**

| MIC result comparing the activity of AgNO₃ and 106, with both having about the same amount of silver. | | | | | | | | | |
|---|---|---|---|---|---|---|---|---|---|
| Sample ID | Conc. of sample | Conc. of sample | Vol. of bacteria | *E. coli* (Day) | | *P*. *aereginousa* (Day) | | *S. aureus* (Day) | |
| | (wt/V%) | (µg/mL) | (µL) | 1 | 2 | 1 | 2 | 1 | 2 |
| AgNO₃ | 0.50 | 3462.35 | 100 | - | - | - | - | - | - |
| | 1DF | 1731.18 | | - | - | - | - | - | - |
| | 2DF | 865.59 | | - | - | - | - | - | - |
| | 3DF | 432.79 | | - | - | - | - | - | - |
| | 4DF | 216.40 | | - | + | - | - | - | + |
| | | | | | | | | | |
| 106 | 1.38 | 3341.48 | 100 | - | - | - | - | - | - |
| | 1DF | 1675.74 | | - | - | - | - | - | - |
| | 2DF | 837.87 | | - | - | - | - | - | - |
| | 3DF | 418.94 | | - | + | - | + | - | + |
| | 4DF | 209.47 | | - | + | - | + | - | + |
| | | | | | | | | | |
| 108 | 0.5 | | 25 | + | | + | | + | |

PC The MIC value was not determined for silver sulfadiazine because of the cloudy nature of the solution, and the concentration of 108 used showed no antimicrobial activity. The dilutions with the least concentration of complex 106 (209 µg/mL) and AgNO₃ (216 µg/mL) in the MIC test was observed to show growth of the same number of colonies of *S*. *aureus* on an agar plate after 24 hrs of incubation. The 25% complex 106/tecophilic fiber mat has the least concentration of Ag, 140 µg/mL (see Table 6), and sustain the release of active silver species that were bioavailable for days. No growth of the organism was observed with the daily increase in the volume of inocula.

**Table 6. Showing details of silver compounds used for the kinetic studies. SSD: silver sulfadiazine 1% cream**

| Sample ID | Wt of Ag compds. used (mg) | Volume of LB Broth (ml) | Amount of Ag in sample (mg) | µg of Ag/mL |
|---|---|---|---|---|
| SSD | 20.00 | 5.00 | 6.05 | 1210.00 |
| AgNO₃ | 12.80 | 5.00 | 8.13 | 1626.00 |
| AgNO₃ | 25.00 | 5.00 | 15.90 | 3176.00 |
| **106**/Tecophilic | | | | |
| (25:75) | 11.30 | 5.00 | 0.73 | 146.00 |
| **106**/Tecophilic | | | | |
| (75:25) | 11.40 | 5.00 | 2.21 | 441.00 |

Thus, the antimicrobial activity of complex 106 was enhanced for a longer period, at a very low concentration of Ag particles by encapsulation in a suitable polymeric fiber. The bactericidal activity of the fiber mat 75% (complex 106/tecophilic) with 424 µg/mL of silver is 8 fold lower in the concentration of Ag than AgNO₃ (3176 µg/mL) and showed not only a kill rate as fast as silver nitrate, but also retained the original color of the LB broth, a clear yellow solution unlike silver nitrate which stains and changed the LB broth color to dark brown. The silver-sulfadiazine cream did not readily dissolve in the aqueous LB broth, thus affecting the rate of its bactericidal activity.

The antimicrobial activity of the fiber mat encapsulating complex 106 can be considered to be a combination of active silver species, which may include AgCl₂ ions, clusters of Ag⁺ ions, AgCl and free Ag⁺ ions. Theoretically, the slow release of the active silver particles in the solution leads to the quick formation of silver chloride. The presence of more chloride anion as the major counter ion will further result in the formation of negatively charged [Ag_{y}Clₓ]ⁿ⁻ ion species (where y = 1, 2, 3...etc; x = 2, 3....(y+1); n = x-1). The anionic silver complexes of the type [AgI₃]²⁻, [Ag₂I₄]²⁻, [Ag₄I₈]⁴⁻ and [Ag₄I₆]²⁻ have been formed. The formation of anionic silver chloride species may not be limited to the leached aggregates of silver particles in the solution, but may also be found on the surface of the fiber mats as shown in the SEM images of Figure 8. Anionic silver dichloride is known to be soluble in an aqueous media and thus will be bioavailable. It has been reported that anionic silver halides are toxic to both sensitive and resistance strain bacteria. The adsorbed active silver species on the network of fibers in the mat is an advantage the fiber mat has to increase the surface area of the active silver species over the conventional use of aqueous silver ions. This mechanism might have accounted for the effective bactericidal activity of the fiber mat in an aqueous media, even at such a low concentration of silver compared to the un-encapsulated form of complex 106. Although complex 106 is sparingly soluble in water, its quick decomposition has been observed to occur in aqueous media. Thus, the bactericidal activity of complex 106 is reduced due to poor availability of active silver species in the LB broth media, which might be due to the formation of secondary silver compound especially AgCl.

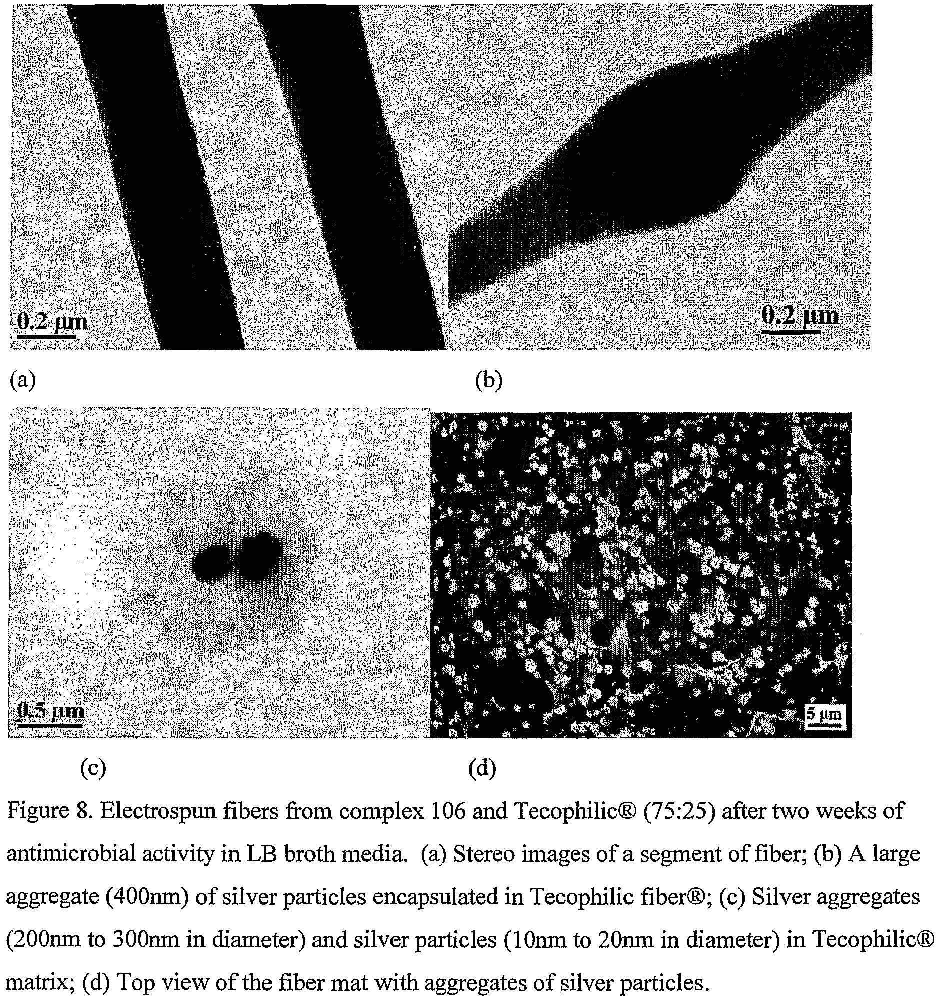

### Acute Toxicity Assessment

The LD 50 assessment was done by intravenous administration of 108, dissolved in a buffered saline solution, via the tail of rats. Adult rats were used with an average weight of 500 g. Progressive administration of 0.3 ml of the dose (5 mg, 50 mg) was done weekly. The rats were carefully examined for the dose-response effect. Death occurred 10 minutes after administrating 50 mg or 108, when 50% % of the rats showed powerful convulsion before death. Autopsy report showed pulmonary hemorrhage and hemorrhage in the brain of the dead rats, a diagnosis of stroke. The surviving rats were observed to lose weight, with a drastic loss in appetite, and low urine out put. The LD 50 assessment was found to be 100 mg/Kg of rat.

The synthesis of 108 with functionalized groups aids in tailoring the encapsulation of the silver(I) imidazole cyclophane gem diol into a nanofiber. The fiber mat has been shown to have improved the antimicrobial activity of the silver(I)-n-heterocyclic carbene complexes on the inoculum, with a faster kill rate than silvadene in an LB broth medium at a concentration 8 fold lower than silvadene. The encapsulation of the silver n-heterocyclic carbene complexes increases the bio-availability of active silver species and also reduces the amount of silver used. Encapsulated silver(I) carbene complexes in nano-fibers has been demonstrated to be a promising material for sustained and effective delivery of silver ions over a longer period of time with maximum bactericidal activity than supplying silver in an aqueous form. The amount of silver required for antimicrobial activity is reduced with this technique of encapsulation compared to the un-encapsulated form, which often is related to the amount of silver in 0.5% silver nitrate. Furthermore, the ability of the fiber mat to retain the original color of the LB broth is a major cosmetic plus. The assessment of the acute toxicity of the ligand on rats showed an LD50 of 100mg/kg of rat, a value considered to be moderately toxic.

In addition to useful antimicrobial, or antibacterial, properties, it is believed that the present invention can inhibit fungal growth, and also viral growth. The compositions of matter and methods of the present invention also contemplate delivery of Silver to locations via any known vehicle, including, but not limited to, inhalation through the lungs, direct application of a liquid to an eye, or any other type of topical application.

### GENERAL EXPERIMENTAL

Silver (I) oxide, silver sulfadiazine and 1, 3-dichloroacetone where purchased from Aldrich. Acetone, acetonitrile, methanol, ethanol, ammonium hexafluorophosphate, and organisms; *S*. *cerevisiae* (ATCC 2601), *C*. *albicans* (ATCC 10231), *A*. *niger* (ATCC 16404), *E*. *coli* (ATCC 8739), *P. aeruginosa* (ATCC 9027), *S*. *aureus* (ATCC 6538) were purchased from Fisher. All reagents were used without further purification. Infrared spectra were recorded on Nicolet Nexus 870 FT-IR spectrometer. The ¹H and ¹³C NMR data was recorded on a Varian Gemini 300 MHz instrument, and the spectra obtained were referenced to the deuterated solvents. Mass spectroscopy data were recorded on an ESI-QIT Esquire-EC with a positive ion polarity. The TEM images were recorded on FEI TE CNAI-12 transmission electron microscope (TEM) at 120KV.

### Synthesis of the imidazolium cyclophane gem-diol dichloride

A solution containing 0.24 g (1.0 mmol) of 2,6-bis(imidazolemethyl)pyridine and 0.254g (2.0 mmol) 1,3-dichloroacetone in 60 ml of acetonitrile was stirred at 75 °C for 8 h to obtain 108 as a brown solid on filtration. Yield: 0.9 mmol, 89.6%. Colorless crystals of the PF₆ salt of 108 were obtained by slow evaporation from acetonitrile/water. Mp: 175-178 °C. ¹H NMR (300 MHz, DMSO-*d*₆): δ 4.68 (s, 4 H, CH₂C(OH)₂CH₂), 5.67 (s, 4 H, CH₂), 7.40, (s, 2 H, NC(H)CH), 7.47 (d, 2 H, J = 7.8 Hz, m-pyr), 7.65 (s, 2 H, C(OH)₂), 7.89 (s, 2 H, NCHC(H)), 7.94 (t, 1H, J = 7.8 Hz, p-pyr), 9.34 (s, 2 H, NC(H)N). ¹³C NMR (75 MHz, DMSO-*d*₆): δ 51.8, 55.2, 91.1, 120.5, 122.0, 123.9, 138.0, 138.8, 152.6. ESI-MS m/z: 384 [M²⁺2Cl⁻], 348 [M²⁺Cl⁻]. FT IR (Nujol, cm⁻¹): 3387, 3105, 1597, 1564, 1439, 1346, 1171, 1085, 996, 755. Anal. Calcd: C, 48.54; H, 4.41; N, 16.94; Cl, 17.13. Found: C, 48.33; H, 4.32; N, 16.71; Cl, 16.76.

### Synthesis of the dinuclear silver carbene cyclophane gem-diol hydroxide

The combination of 0.232 g (1.0 mmol) silver(I)oxide and 0.366g (0.9 mmol) of 108 in 70 ml methanol was stirred at room temperature for 50 minutes. The filtrate was concentrated to obtain complex 106 as a yellow solid. Single crystals of complex 106 were obtained from ethanol, containing a spike of carbonate, by slow diffusion.
Yield: 0.618g, 0.738 mmol, 82%. Mp: 202-204 °C. ESI-MS m/z: 400[0.5M²⁺], 801[2M⁺], 837[2M⁺2OH⁻]. FTIR (Nujol, cm⁻¹): 3415, 3105, 1596, 1564, 1439, 1344, 1169, 1084, 1028, 996, 758. ¹³C NMR (75 MHz, DMSO-*d*₆): δ 48.6, 51.1, 53.8, 92.1, 119.9 (J = 1.4 Hz), 121.6, 128.6, 137.8(J = 2.4 Hz), 154.2, 184.9 (Jcarbene-Ag = 211 Hz). Anal. Calcd: Ag, 24.54; C, 43.79; H, 4.20; N, 15.24. Found: C, 43.15; H, 4.22; N, 14.89.

### Electrospun Fiber

Tecophilic® was dissolved in a mixture of ethanol and tetrahydrofuran at a ratio of 9 to 1. A solution of complex 106 in ethanol was mixed with a pre-made solution of Tecophilic®. Solutions with different weight ratios between complex 106 and Tecophilic® were prepared. The ratios were 0/100, 25/75 and 75/25. The solutions of complex 106 and Tecophilic® were held in a pipette. An electrical potential difference of 15KV was applied between the surfaces of the solution drop to the grounded collector, a distance of about 20 cm. Transmission electron microscopy (TEM) and scanning electron microscopy (SEM) were used to characterize the as-spun fibers and fibers exposed to water.

### Antimicrobial Test

Sterilized LB Broth was measured (5 mL) into a sterile tube. A loopful of stationary phase cultured microorganism (*E coli, P. aeruginosa, S. aureus*) was introduced into the tube containing the LB Broth solution. The mixture was cultured overnight, at 35°C in a shaking incubator. The same procedure was done with stationary phased cultured fungi (*C*. *albican, S. cerevisae, A. niger*) and incubated without shaking at room temperature for 72 hrs.

### Fiber Mat Testing

A constant volume (25µL) of the freshly grown organism was placed on an LB agar plate and grown to obtain a lawn of the organism. A fiber mat (2.0 cm x 2.0 cm) of complex 106 and pure tecophilic was placed on a lawn of bacteria (*E. coli, P. aeruginosa, S. aureus*) of an LB agar plate and incubated overnight at 35 °C. The bactericidal activity was observed by visual inspection of growth and no growth in and around the area of the fiber mat. About the same dimension of the fiber mat was placed on a lawn of fungi (*C*. *albican, S. cerevisae, A. niger*) and incubated at room temperature for 48 hrs. The diameter of the clear zone was measured.

### Minimum Inhibitory Concentration (MIC) Test.

Serial dilutions were made to obtain a range of concentrations by transferring 1 mL of freshly prepared stock solution of the silver compounds (with the same amount of silver particles) into a sterile culture tube containing 2 mL of LB broth, marked A. 1 mL of well mixed solution of A was transferred to culture tube B containing LB broth. The same procedure was repeated to obtain the dilute solution for tube C, D and E. The MIC was determined by visual inspection of growth /no-growth of the above concentrations of the silver compounds marked A-E inoculated with 25 µl of the organisms. After incubation at 35 °C overnight with no growth of organism, an additional 80 µL of freshly grown organisms was added to each of the culture on the second day and incubated at the same temperature.

### Kinetic Test of Bactericidal Activity:

Equal volume (5 mL) of LB broth were measured into sterile culture tubes and inoculated with 100 µL of *S. aureus* to each tube containing silver nitrate (12.8 mg, 25 mg), silver sulfadiazine (20 mg), 11.3mg complex 106/tecophilic (25:75) and 11.4 mg complex 106/tecophilic (75:25) fiber mats. The mixtures were incubated at 35 °C and the bactericidal activity was checked over a range of time by streaking one loopful of each mixture on an agar plate. The agar plate was then incubated at 37 °C overnight and the numbers of colonies of organism formed counted. The same procedure was repeated using 100 µL *P. aeruginosa.*

### Animal studies

Male Sprague Dawley (Harlen Sprague Dawley, Indianapolis, IN) adult rats (400-500g body weight) were housed in the university of Akron animal facility. Temperature and humidity were held constant, and the light/dark cycle was 6.00 am - 6.00 pm: light, 6.00 pm -6.00 am: dark. Food (Lab diet 5P00, Prolab, PMI nutrition, Intl., Bretwood, Mo.) and water were provided ad libitim. Animals were anesthetized with ether in order to inject the compound into the tail vein, using a 27 gauge syringe needle in a volume of 0.3ml sterile saline. The dosages for the ligand were 5mg and 50mg. At the end dosages of the experiment, animals were terminated and the liver, lung, kidney and heart tissues were removed and frozen at -70 °C. Urine samples were collected daily for later examination of the compound distribution. These studies were approved by the University of Akron Institutional Animal Care and Use Committee (IACUC).

### X-ray Crystallographic Structure Determination.

Crystal data and structure refinement parameters contained in the supporting information. Crystals of 108 and complex 106 were each coated in paraffin oil, mounted on kyro loop, and placed on a goniometer under a stream of nitrogen. X-ray data were collected at a temperature of 100 K on a Brucker Apex CCD diffractometer using Mo Kα radiation (λ = 0.71073 Å). Intensity data were intergrated using SAINT software, and an empirical absorption correction was applied using SADABS. Structures 108 and complex 106 were solved by direct methods and refined using full-matrix least square procedures. All non-hydrogen atoms were refined with anisotropic displacement.

It should be evident that the present invention is highly effective in providing a method of inhibiting microbial growth by administration of a N-functionalized silver carbene complex.

## Claims

1. A pharmaceutical composition of a silver complex of an N-heterocyclic carbene **characterized in that** the silver complex of an N-heterocyclic carbene is represented by the formula: 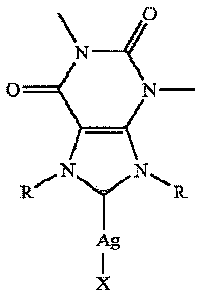 where each R is independently selected from a hydrogen, a C₁ to C₁₂ alkyl, a C₁ to C₁₂ substituted alkyl, a C₁ to C₁₂ alkoxy, a cycloalkyl having up to 12 carbon atoms, a substituted cycloalkyl having up to 12 carbon atoms, an alkenyl having up to 12 carbon atoms, a cycloalkenyl having up to 12 carbon atoms, a substituted cycloalkenyl having up to 12 carbon atoms, an alkynyl having up to 12 carbon atoms, an aryl having up to 12 carbon atoms, a substituted aryl having up to 12 carbon atoms, an arylalkyl having up to 12 carbon atoms, an alkylamine having up to 12 carbon atoms, an substituted alkylamine having up to 12 carbon atoms, an alkylpentose phosphate having up to 12 carbon atoms, a phenol having up to 12 carbon atoms, an ester having up to 12 carbon atoms, an alkylaryl having up to 12 carbon atoms, a pyrrole having up to 12 carbon atoms, a pyridine having up to 12 carbon atoms, or a thiophene having up to 12 carbon atoms, and where X is selected from a halide, a carbonate group, an acetate group, a phosphate group, a hexafluorophosphate group, a tetrafluoroborate group, a nitrate group, a methylsulfate group, a hydroxide or sulfate.

2. The pharmaceutical composition of claim 1, wherein the silver complex of an N-heterocyclic carbene is represented by the following formula:

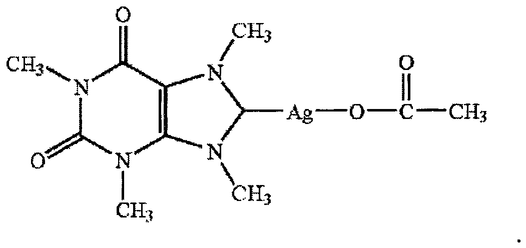

3. The pharmaceutical composition as defined in claim 1 or 2 for use in the treatment of infections.

4. The pharmaceutical composition of claim 3 for use in the treatment of an infection which is due to microbial, fungal, or viral growth.

5. A nanofiber comprising:
a fiber-forming material; and
the pharmaceutical composition as defined in claim 1 or 2.

6. A wound dressing comprising the nanofiber of claim 5.

## Patentansprüche

1. Pharmazeutische Zusammensetzung eines Silberkomplexes eines N-heterocyclischen Carbens, **dadurch gekennzeichnet, dass** der Silberkomplex des N-heterocyclischen Carbens durch die folgende Formel dargestellt wird: 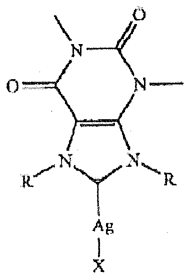 wobei R jeweils unabhängig aus Wasserstoff, C₁- bis C₁₂-Alkyl, substituiertem C₁- bis C₁₂-Alkyl, C₁- bis C₁₂-Alkoxy, Cycloalkyl mit bis zu 12 Kohlenstoffatomen, substituiertem Cycloalkyl mit bis zu 12 Kohlenstoffatomen, Alkenyl mit bis zu 12 Kohlenstoffatomen, Cycloalkenyl mit bis zu 12 Kohlenstoffatomen, substituiertem Cycloalkenyl mit bis zu 12 Kohlenstoffatomen, Alkinyl mit bis zu 12 Kohlenstoffatomen, Aryl mit bis zu 12 Kohlenstoffatomen, substituiertem Aryl mit bis zu 12 Kohlenstoffatomen, Arylalkyl mit bis zu 12 Kohlenstoffatomen, einem Alkylamin mit bis zu 12 Kohlenstoffatomen, einem substituierten Alkylamin mit bis zu 12 Kohlenstoffatomen, einem Alkylpentosephosphat mit bis zu 12 Kohlenstoffatomen, einem Phenol mit bis zu 12 Kohlenstoffatomen, einem Ester mit bis zu 12 Kohlenstoffatomen, einem Alkylaryl mit bis zu 12 Kohlenstoffatomen, einem Pyrrol mit bis zu 12 Kohlenstoffatomen, einem Pyridin mit bis zu 12 Kohlenstoffatomen oder einem Thiophen mit bis zu 12 Kohlenstoffatomen ausgewählt ist und wobei X aus einem Halogenid, einer Carbonatgruppe, einer Acetatgruppe, einer Phosphatgruppe, einer Hexafluorophosphatgruppe, einer Tetrafluoroboratgruppe, einer Nitratgruppe, einer Methylsulfatgruppe, einem Hydroxid oder Sulfat ausgewählt ist.

2. Pharmazeutische Zusammensetzung gemäß Anspruch 1, wobei der Silberkomplex des N-heterocyclischen Carbens durch die folgende Formel dargestellt wird:

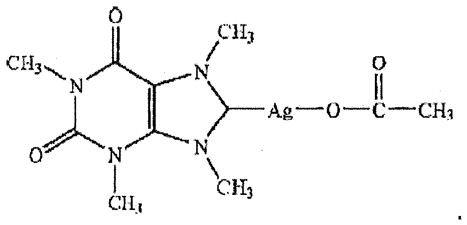

3. Pharmazeutische Zusammensetzung gemäß Anspruch 1 oder 2 zur Verwendung bei der Behandlung von Infektionen.

4. Pharmazeutische Zusammensetzung gemäß Anspruch 3 zur Verwendung bei der Behandlung einer Infektion, die auf Mikroben-, Pilz- oder Virenwachstum zurückgeht.

5. Nanofaser, umfassend:
ein faserbildendes Material; und
die pharmazeutische Zusammensetzung gemäß Anspruch 1 oder 2.

6. Wundverband, der die Nanofaser gemäß Anspruch 5 umfasst.

## Revendications

1. Composition pharmaceutique d'un complexe d'argent d'un carbène N-hétérocyclique **caractérisée en ce que** le complexe d'argent d'un carbène N-hétérocyclique est représenté par la formule : 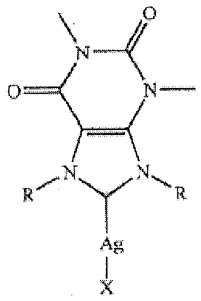 dans laquelle chaque R est indépendamment choisi parmi un atome d'hydrogène, un alkyle en C₁ à C₁₂, un alkyle en C₁ à C₁₂ substitué, un alcoxy en C₁ à C₁₂, un cycloalkyle ayant jusqu'à 12 atomes de carbone, un cycloalkyle substitué ayant jusqu'à 12 atomes de carbone, un alcényle ayant jusqu'à 12 atomes de carbone, un cycloalcényle ayant jusqu'à 12 atomes de carbone, un cycloalcényle substitué ayant jusqu'à 12 atomes de carbone, un alcynyle ayant jusqu'à 12 atomes de carbone, un aryle ayant jusqu'à 12 atomes de carbone, un aryle substitué ayant jusqu'à 12 atomes de carbone, un arylalkyle ayant jusqu'à 12 atomes de carbone, une alkylamine ayant jusqu'à 12 atomes de carbone, une alkylamine substituée ayant jusqu'à 12 atomes de carbone, un alkylpentose phosphate ayant jusqu'à 12 atomes de carbone, un phénol ayant jusqu'à 12 atomes de carbone, un ester ayant jusqu'à 12 atomes de carbone, un alkylaryle ayant jusqu'à 12 atomes de carbone, un pyrrole ayant jusqu'à 12 atomes de carbone, une pyridine ayant jusqu'à 12 atomes de carbone, ou un thiophène ayant jusqu'à 12 atomes de carbone, et dans laquelle X est choisi parmi un halogénure, un groupe carbonate, un groupe acétate, un groupe phosphate, un groupe hexafluorophosphate, un groupe tétrafluoroborate, un groupe nitrate, un groupe méthylsulfate, un hydroxyde ou un sulfate.

2. Composition pharmaceutique selon la revendication 1, dans laquelle le complexe d'argent d'un carbène N-hétérocyclique est représenté par la formule suivante :

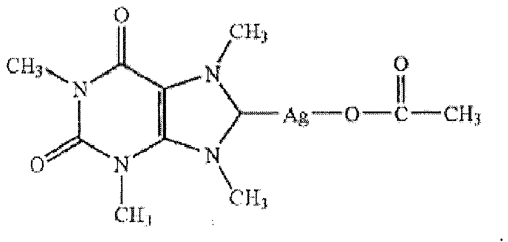

3. Composition pharmaceutique telle que définie dans la revendication 1 ou 2, destinée à être utilisée dans le traitement des infections.

4. Composition pharmaceutique selon la revendication 3, destinée à être utilisée dans le traitement d'une infection due à une croissance microbienne, fongique ou virale.

5. Nanofibre comprenant :
une matière fibrogène et
la composition pharmaceutique selon la revendication 1 ou 2.

6. Pansement comprenant la nanofibre selon la revendication 5.
